# EUROPEAN PATENT APPLICATION

(11) **EP 3 421 061 A1**
(43) Date of publication of application: **02.01.2019**
(21) Application number: 17178458.0
(22) Date of filing: 28.06.2017
(51) Int. Cl.: A61M 1/34, A61M 1/36, A61M 5/14, A61M 39/10

(54) **ANOTHER INSERT PIECE FOR A BLOOD TUBING SET TO PROMOTE MIXING AN INFUSION SOLUTION WITH A FURTHER FLUID**

(71) Applicant: Fresenius Medical Care Deutschland GmbH, 61352 Bad Homburg (DE)
(72) Inventor: Benelli, Davide Maria, 26013 Crema (IT); Fini, Massimo, 26010 Casaletto Vaprio (IT); Reiter, Reinhold, 26013 Crema (IT)
(74) Representative: Bobbert & Partner Patentanwälte PartmbB

(57) **Abstract**

The present invention relates to an insert piece (100) for a blood tubing set (200), comprising blood tubing set (200), comprising at least, respectively, a first connection site (101) for connecting a first tubing portion (205a) of the blood tubing set (200) to the insert piece (100); a second connection site (103) for connecting a second tubing portion (205b) of the blood tubing set (200) to the insert piece (100); a third connection site (105) for connecting a third tubing portion (209) of the blood tubing set (200) to the insert piece (100); a first main line (107a) for conducting a first liquid, preferably blood, through the insert piece (100), wherein the first main line (107a) is in fluid communication with the first connection site (101) and with the second connection site (103); a second main line (107b) for conducting a first liquid, preferably blood, through the insert piece (100), wherein the second main line (107b) is in fluid communication with the first connection site (101) and with the second connection site (103); a secondary line (111) for conducting a second liquid, preferably an infusion, into at least one of the first main line (107a); the second main line (107b); the secondary line (111), and a connection portion (109) which connects both main lines (107a, 107b) to each other or to the second connection site (103); wherein the secondary line (111) is in fluid communication with the third connection site (105); the first connection site (101) comprises a perfusable lumen having a first cross section area; the second connection site (103) comprises a perfusable lumen having a second cross section area; and the first main line (107a) and/or the second main line (107b) comprises a perfusable lumen having a third cross section area.

## Description

The present invention relates to an insertion piece according to the preamble of claim 1, an extracorporeal blood tubing set according to the preamble of claim 11 and a blood treatment apparatus according to the preamble of claim 15.

During the extracorporeal blood treatment, infusion solutions or medicaments are mostly infused through the extracorporeal blood circuit, i.e., the utilized blood tubing system. Depending on the type of the infusion solution, a fast mixing of the infusion solution with the blood may be favorable or desirable.

For example, anticoagulation infusion solutions are regularly infused in an extracorporeal blood treatment to prevent a possible occlusion of the extracorporeal blood circuit.

Two methods are mainly used for this purpose; the system and the regional anticoagulation. A citrate solution, which complexes calcium, and thus suppresses the blood coagulation, is mostly used as an anticoagulant in the regional anticoagulation. Prior to returning blood to the patient, an additional calcium must be substituted, since too-low calcium concentrations influence nerves and muscles, the blood coagulation and functions of lung, heart and kidneys. In the regional anticoagulation, a calcium-containing solution, by which the physiological calcium concentration in the system blood may be maintained, is therefore added to the blood before reinfusing it into the patient.

Infusing infusion solutions or medicaments into the tubing set takes place usually through addition sites with a T-form, the so-called Tees. Laminar flow conditions predominate in these Tees due to the circular cross section and the smooth inner wall at the addition site. In addition, the flow rates of the infused solutions are low in comparison with the blood flow.

The largely laminar flow conditions and the low flow rates of the infusion solutions or medicaments flowing into the blood stream may delay or slow down the mixing of the blood with the added infusion. This slow mixing of both liquids at the addition site is undesired, especially when adding the calcium solution to blood, and may lead to clot problems at the addition site, due to the punctually continued higher calcium concentrations in blood. To prevent that, a fast and homogenous mixing of the added calcium solution with the blood is desirable.

The same problem may however also occur when mixing other liquids, e.g. medicaments, the fast mixing of which may as well be advantageous.

This problem is known in the prior art. In order to solve it, special addition sites, which have means for generating turbulences, have been described.

For example, an insert piece in form of a T-piece having a spiral structure for a blood tubing set is thus described in WO 2014/026771 A1. The spiral structure serves generating turbulences in the area of the infusion site. The turbulences serve or contribute to a better mixing of the fluids which are brought together.

Furthermore, there are solutions which generate a pulsed infusion flow of the infusion solution into the blood through intermittent operation of an infusion pump.

An object of the present invention may be to propose a further solution for promoting a mixing of an infusion solution with a further fluid, e.g. blood.

The object may be solved by an insert piece having the features of claim 1, an extracorporeal blood tubing set having the features of claim 11 and a blood treatment apparatus having the features of claim 15.

The insert piece according to the present invention is intended to be inserted into a blood tubing set (or into a blood tubing system or an extracorporeal blood circuit) or to be part thereof, respectively. The insert piece comprises at least a first connection site for connecting a first tubing portion of the blood tubing set to the insert piece. It comprises a second connection site for connecting a second tubing portion of the blood tubing set to the insert piece. It comprises a third connection site for connecting a third tubing portion of the blood tubing set to the insert piece. It comprises a first main line for conducting a first liquid, preferably blood, through the insert piece. The first main line is in fluid communication with at least the first connection site and with the second connection site or with a lumen surrounded or formed by the first connection site and/or the second connection site.

The insert piece comprises at least a second main line for conducting the first liquid, preferably blood, through the insert piece. Like the first main line, the second main line is also in fluid communication with the first connection site and with the second connection site.

The insert piece comprises at least one secondary line for conducting, directly or indirectly, a second liquid, preferably an infusion solution, into an element from the group consisting of the first main line, the second main line and a connection portion which connects both the first main line and the second main line to each other and/or to the second connection site.

The secondary line is in fluid communication with the third connection site or with a lumen surrounded or delimited by it.

The first connection site comprises a perfusable or flow-through lumen having a first cross section area. The second connection site comprises a perfusable or flow-through lumen having a second cross section area. At least one of the first main line and the second main line comprises a perfusable lumen having a third cross section area. The secondary line comprises a lumen portion. The lumen portion comprises at least one opening or outlet opening for the second fluid.

The blood tubing set, extracorporeal blood circuit or blood tubing system comprises at least one insert piece according to the present invention.

In some embodiments, the insert piece according to the present invention is pressed or interposed between tubing portions of the blood tubing set or it is integrally manufactured therewith.

In some embodiments, the insert piece according to the present invention is firmly connected to at least one of the tubing portions of the blood tubing set, in others, it is releasably connected.

The blood treatment apparatus is connected to at least one blood tubing set according to the present invention.

All embodiments mentioned herein may be exemplary embodiments according to the present invention.

Embodiments mentioned herein may encompass one or several of the aforementioned or following features in any arbitrary combination unless the skilled person finds a particular combination as technically impossible. Embodiments are moreover subject-matter of the dependent claims.

Whenever numerical words are mentioned herein, the person skilled in the art shall recognize or understand them as indications of numerical lower limits. Unless it leads the person skilled in the art to an evident contradiction, the person skilled in the art shall comprehend the specification for example of "one" encompassing "at least one". This understanding is also equally encompassed by the present invention as the interpretation that a numeric word, for example, "one" may alternatively mean "exactly one", wherever this is evidently technically possible for the person skilled in the art. Both are encompassed by the present invention and apply herein to all used numerical words.

Spatial information given herein such as "top", "bottom" etc. refer in case of doubt to the illustration as seen in the accompanying figures.

In certain embodiments, the first connection site, the second connection site and/or the connection portion comprise, respectively, a mostly cylindrical inner wall or a section having a cylindrical inner wall.

In certain embodiments, the insert piece is manufactured integrally with the blood tubing set. All or some of the sections of the insert piece denoted herein as connection sites are in such embodiments passage sections between insert piece and adjacent or continuing tubing portions.

In certain embodiments, the secondary line is connected in fluid communication with, or comprises, a source for the infusion solution.

In certain embodiments, the infusion solution is a calcium solution or comprises a calcium solution. The present invention is however not limited to using a calcium-containing solution. Other medicament solutions are encompassed by the present invention as well. They include, in particular, citrate solution.

In certain embodiments, the third cross section area is, as a maximum, half, or substantially half, as large, as the first cross section area and/or the second cross section area. "Substantially half as large" may refer to or take on a value between 35% and 65%. All intermediate values and in particular each integer percentage value (36%, 37%, 38%, etc.) are included or contemplated as well.

In certain embodiments, the cross section area of the first main line and the cross section area of the second main line taken together or their sum are smaller than the cross section of the first connection site, the second connection site and/or the cross section of tube section of the blood tube set connected to the insert piece. With this design, the flow of the first liquid can be speed up within the first and/or the second main line which in turn contributes to generating turbulences and to prevent poor mixing.

The entrance into the first main line and/or into the second main line can optionally be tapered or result in another way in a decreased cross section. A such amended cross section may advantageously cause disturbance in the flow of the first liquid and turbulences. Turbulence, in turn, may contribute to an enhanced mixing of the first and the second liquids further downstream. The same applies optionally to the exit from the first main line and/or from the second main line.

Also, the cross sections need not to be increased or decreased by deviations from, e. g., a circular cross section or need not be homogeneously altered along their entire circumference. Rather, amending parts of the circumference only may already lead to the desired turbulences.

In certain embodiments, a baffle or deflector element is provided in the area of the connection portion and in the interior of the latter. This element is arranged such that a second liquid flowing out of the outlet opening or opening of the secondary line is limited in its radial movement or in its movement in the outlet direction.

In certain embodiments, the baffle or deflector element is not the inner wall of the main line, for example in the area of the first connection site, of the second connection site or the connection portion.

In certain embodiments, an addition line is connected in fluid communication to a blood return line and/or to a blood withdrawal line of the blood tubing set via the secondary line of or connected to the insert piece.

In certain embodiments, the blood tubing set is suitable and/or provided for executing a regional anticoagulation.

In certain embodiments, the blood tubing set is suitable and/or provided for executing a hemodialysis, a hemofiltration or a hemodiafiltration or a plasmapheresis treatment or a whole blood adsorption treatment.

In some embodiments, the connection portion is arranged - in flow direction of the first liquid - between the first main line and/or the second main line on the one hand and the second and/or the third connection site on the other. Hence, it may be arranged downstream to the first and/or the second main line. It may be arranged up-stream to the second and/or the third connection site.

In some embodiments, the connection portion comprises, in flow direction towards the second connection site or up-stream to the second connection site, a cross section, in particular a conical one, which widens, increases or enlarges along the flow direction.

In some embodiments, there is a through-opening of the inset piece arranged between or delimited or framed by sections of the first main line and sections of the second main line.

In some embodiments, the through-opening has a round, oval or oblong hole form or shape. Alternatively, it comprises a section which has a round, oval or oblong hole form.

In some embodiments, the insert piece consists of two or at least two components, which are welded and/or glued with or to each other. For example, the tubes may be glued or welded to the insert piece. Also, the insert piece may consist of, e. g., an upper part and a lower part which are glued one to the other. Alternatively, or in addition, they may be interconnected to each other by other methods such as clamping and the like.

In some embodiments, the insert piece is integrally formed, e. g. by injection molding. The tubes may optionally be clamped and/or glued to the insert piece. This may be the simplest ways for assembling the components to each other. Also, a welding process may be waived this way.

In some embodiments, at least one of the first main line and the second main line, or sections thereof, respectively, are tubes. They may, in particular, be those which are inserted into or integral with tube intakes or connectors which are in fluid communication with the first connection site or with the second connection site, and which are optionally integrally produced therewith.

In some embodiments, at least one of the first main line and the second main line are integral parts of the insert piece or integrally with the latter.

In certain embodiments, the insert piece is made of plastic, preferably injection molded.

In certain embodiments, the insert piece has no three-dimensional spiral structure for generating turbulences, in particular no recessed section in the inner wall of the connection portion.

In some embodiments, the first, the second and/or the third connection site is bonded or connected to a tubing portion, respectively.

In some embodiments, the blood tubing set may comprise at least one of a drip chamber, further insert pieces in form of Tees or injection points, and the like.

When liquids are mentioned herein, it is not to be understood restrictively. The present invention encompasses or contemplates also bringing together other fluids in a wider sense.

Some or all of the embodiments according to the present invention may include one or several of the aforementioned or following advantages.

One advantage is that blood clots resulting from improper mixing of the first and the second fluids may be prevented. Hence, the treatment needs not to be interrupted because of clotting and/or occlusions.

According to another advantage no further devices or method steps are required for ensuring a swirling of the infusion solution within the blood due to the particular shape or design of the insert piece described herein. Nevertheless, an accelerated and reliable mixing of the infusion solution with the blood may be achieved.

An advantage of using the insert piece according to the present invention may further be that the herein described advantages may be achieved without requiring a change in the controlling of the blood treatment apparatus or the pump for the infusion solution. Rather, the structure of the insert piece effects an advantageous distribution of the second fluid into the first fluid without requiring, e. g., a pulsating addition as described in, e. g., DE 10 2013 011 010 A1. The effect according to the present invention may be achieved by the geometry of the insert piece described herein alone. In particular, no amendments to the controlling of the infusion pump are required. Its conveying characteristics may be maintained.

This advantageously implies that no structural or other type of adjustment of the already purchased blood treatment apparatus, e. g. a software adjustment or modification, is required. The implementation of the present invention may consist of using the insert pieces or blood tubing sets as set forth herein. Switching to such blood tubing sets is also easy to accomplish since they are anyhow single-use articles or disposables.

The effect of the insert piece according to the present invention is independent of parameters of the blood treatment. If for example the conveying rate of the blood pump and the conveying rate of the pump for the infusion solution being in line with the former are changed, then the effect principle or the effect of the insert piece according to the present invention remains unchanged or undisturbed.

Finally, the insert piece convinces or impresses due to its extremely simple and, when desired, even symmetrical design. It can be produced by most simple injection molding tools which may reduce the total costs of its production significantly.

The present invention is exemplarily explained in the following, regarding the accompanying drawings in which identical reference numerals denote the same or similar elements. The following applies in the partially highly simplified figures:
- Fig. 1: shows the insert piece in a first exemplary embodiment;
- Fig. 2: shows the insert piece of Fig. 1 in a first cut in the longitudinal direction;
- Fig. 3: shows the insert piece of Fig. 1 in a second cut in the longitudinal direction;
- Fig. 4: shows the insert piece in a second exemplary embodiment;
- Fig. 5: shows the insert piece similar to that of Fig. 4 in a first cut in the longitudinal direction in a plan view;
- Fig. 6: shows the lower part of the insert piece of Fig. 4 in a second cut in the longitudinal direction in perspective from above;
- Fig. 7: shows the upper part of the insert piece of Fig. 4 in a second cut in the longitudinal in perspective direction from below;
- Fig. 8: shows the insert piece according to a third exemplary embodiment in a first cut in the longitudinal direction;
- Fig. 9: shows a partly cut insert piece according to a fourth exemplary embodiment;
- Fig. 10: shows the insert piece of Fig. 9 in a longitudinal section; and
- Fig. 11: shows a blood tubing set according to the present invention having an insert piece according to the present invention.

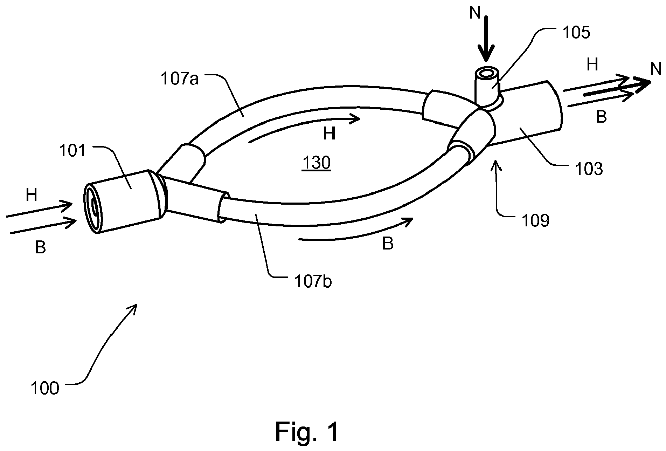

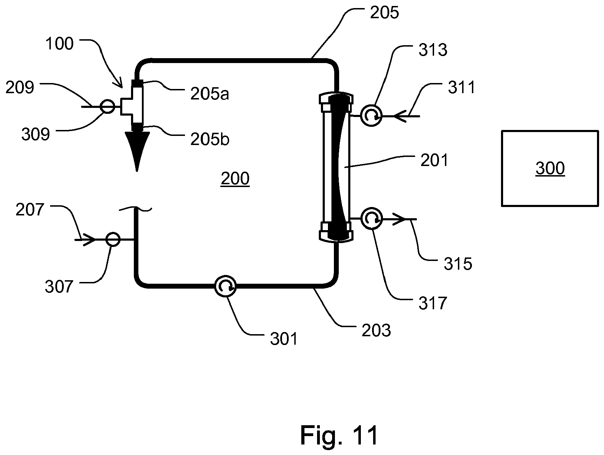

**Fig. 1** shows an insert piece 100 in a first exemplary embodiment according to the present invention for a blood tubing set 200, not shown in Fig.1 (see however Fig. 11).

The insert piece 100 comprises a first connection site 101 by means of which a first tubing portion 205a (see Fig. 11) of the blood tubing set 200 may be connected to the insert piece 100.

The insert piece 100 comprises a second connection site 103 by which a second tubing portion 205b of the blood tubing set 200 may be connected to the insert piece 100.

The insert piece 100 comprises a third connection site 105 by which a third tubing portion, here it is a line 209 for calcium solution of the blood tubing set 200 (see Fig. 11), may be connected to the insert piece 100.

The insert piece 100 comprises a first main line 107a for conducting a first liquid, preferably blood, through the insert piece 100. The first main line 107a is in fluid communication with the first connection site 101 and with the second connection site 103. The first liquid may flow in direction of the arrows H into and through the first main line 107a.

The insert piece 100 comprises also at least a second main line 107b (and possible even a third, fourth, and so on line) for conducting the first liquid through the insert piece 100. The second main line 107b is in fluid communication with the first connection site 101 and with the second connection site 103. The first liquid may flow in direction of the arrows B into and through main line 107b.

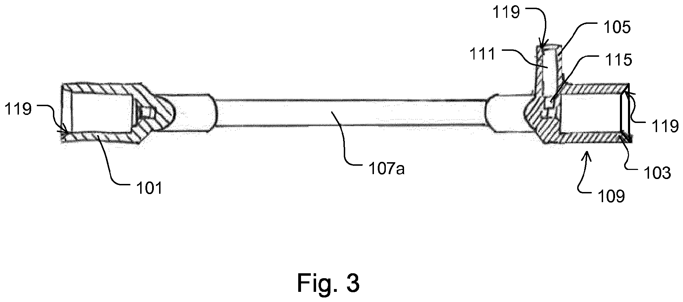

The insert piece 100 comprises an optional junction portion (also referred to as a connection portion herein) 109, in which the first main line 107a, the second main line 107b and a secondary line 111 (see Fig. 3) meet or end. The junction portion 109 may comprise also the second connection site 103 and/or the third connection site 105.

The insert piece 100 comprises a secondary line 111. It serves conducting a second liquid, preferably an infusion solution. The second liquid may flow in direction of the arrow N into the secondary line 111.

The secondary line 111 is in fluid communication with the third connection site 105, and it can, as in Fig. 1, be part of or end with the third connection site 105. It is further in fluid communication with the first and the second main lines 107a, 107b.

The first connection site 101 comprises a perfusable lumen having a first cross section area.

The second connection site 103 comprises a perfusable lumen having a second cross section area.

The first and/or the second main lines 107a, 107b may comprises a perfusable lumen having a third cross section area.

The secondary line 111 comprises a lumen portion which protrudes or opens into the interior of the junction portion 109 and which comprises at least one opening or outlet opening 115. The second liquid may be introduced into a lumen of the junction portion 109 or of another line through the outlet opening 115.

As Fig. 1 further shows, the lumen portion opens into the interior of the junction portion 109.

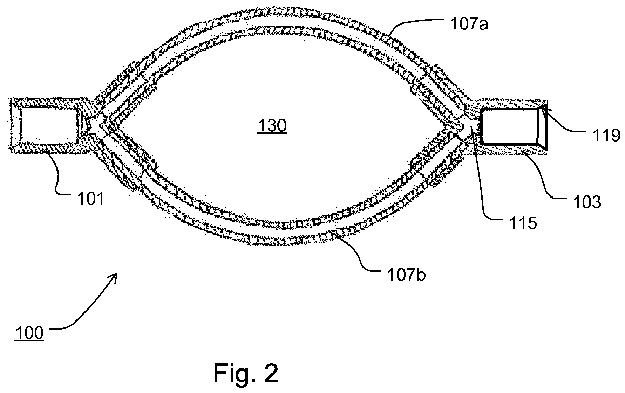

The first connection site 101, the second connection site 103 and/or the third connection site 105 may optionally comprise, respectively, a chamfer 119 of the inner wall or of the outer wall, see Fig. 2 and Fig. 3.

The first and the second main lines 107a, 107b may, as in Fig. 1, delimit an optional through-opening 130 of the insert piece 100.

**Fig. 2** shows the insert piece 100 of Fig. 1 in a first cut in the longitudinal direction in a plan view.

**Fig. 3** shows the insert piece 100 of Fig. 1 in a second cut in the longitudinal direction.

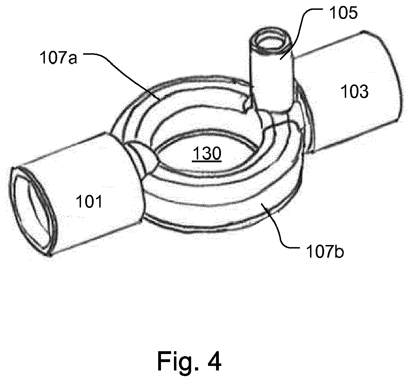

**Fig. 4** shows the insert piece 100 in a second exemplary embodiment.

In contrast to the embodiment shown in Figs. 1 to 3, the first main line 107a and the second main line 107b are integrally formed parts of the insert piece 100 and, hence, optionally made from the same material as, e.g., the first connection site 101 which facilitates the production process of the insert piece 100. The insert piece 100 can be formed, e. g., by injection molding.

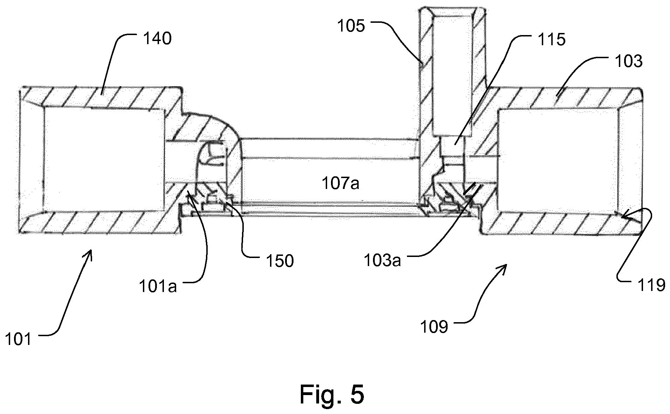

**Fig. 5** shows the insert piece 100 similar to that of Fig. 4 in a first cut in the longitudinal direction.

Reference numeral 101a denotes an entrance into the first main line 107a and/or into the second main line 107b. The entrance 101a can be either part of the first connection site 101 or of the first and/or second main lines 107a, 107b.

Reference numeral 103a denotes an exit from the first main line 107a and/or from the second main line 107b. The exit 103a can be either part of the second connection site 103 or of the first and/or second main lines 107a, 107b.

The presence of an entrance 101a and/or an exit 103a and their designs as discussed above, are, of course, not limited to the second embodiment as shown in Fig. 5. Only, they can be best observed in Fig. 5.

As is exemplarily shown in Fig. 5, the insert piece 100 may comprise or consist of an upper part 140 (shaded in a first manner) and a lower part 150 (shaded differently than the upper part 140) which may be glued one to the other or connected to each other in any other way (such as welding).

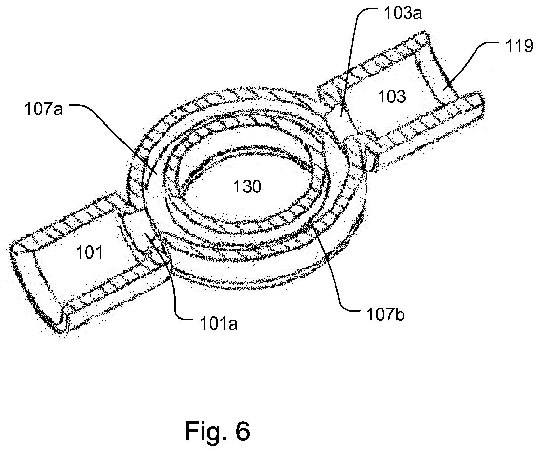

**Fig. 6** shows one part of the insert piece 100 of Fig. 4 in a second cut in the longitudinal direction from above and in slight perspective.

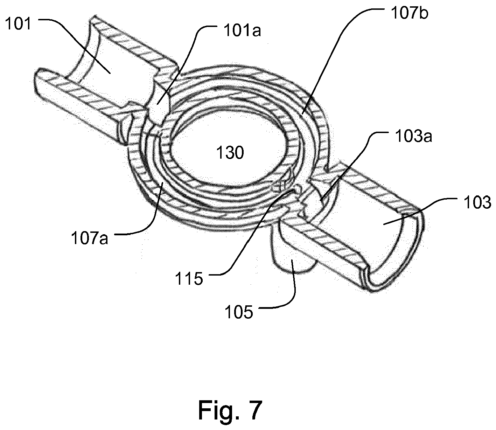

**Fig. 7** shows the other part of the insert piece 100 of Fig. 4 in the second cut in the longitudinal direction from below and in slight perspective.

In the particular embodiment shown in Fig. 7 or in any other embodiment, the cross section area of the first main line 107a and/or the cross section of the second main line 107b is less than half of the cross section area of the first connection site 101 and/or of the second connection site 103.

For example, the first connection site 101 may have a cross section area of 14.51 mm² (at a diameter of 4.3mm) whereas the cross section area of the first main line 107a and/or the cross section of the second main line 107b may be 2.83 mm² (at a diameter of 1.9 mm). Other values for the cross section area of the first main line 107a and/or the cross section of the second main line 107b such as 3.14 mm² (at a diameter of 2.0 mm) are encompassed as well, of course.

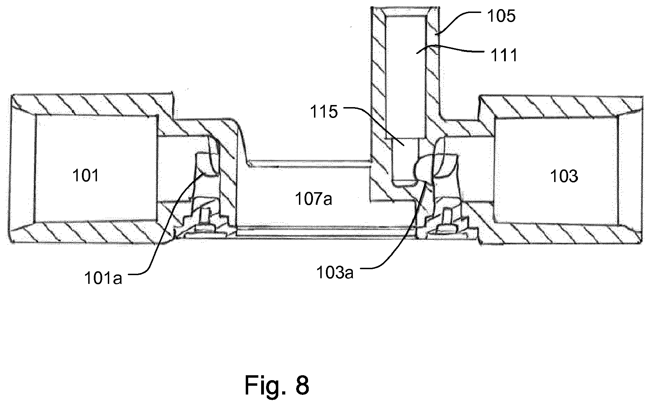

**Fig. 8** shows the insert piece 100 in a third exemplary embodiment in a first cut in the longitudinal direction.

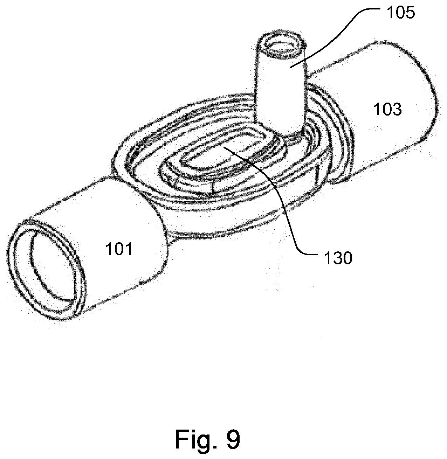

**Fig. 9** shows a partly cut insert piece 100 in a fourth exemplary embodiment.

In contrast to the insert piece 100 of, e. g., Figs. 1 to 3, the through-opening 130 is oval in shape.

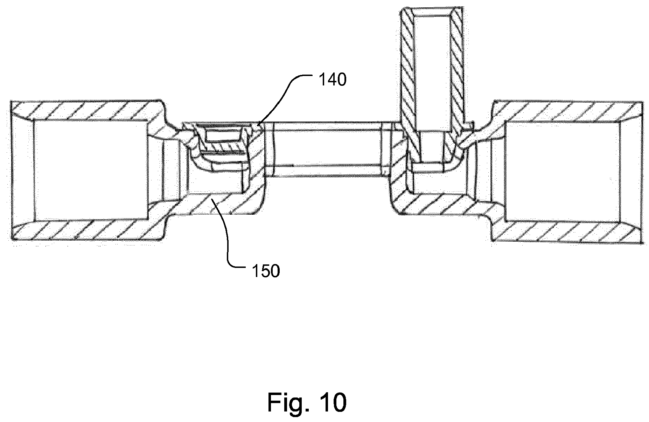

**Fig. 10** shows the insert piece 100 of Fig. 9 in a longitudinal section.

**Fig. 11** shows an exemplary embodiment of an optional basic arrangement of an insert piece 100 as described herein in an extracorporeal blood tubing set 200 according to the present invention, the latter being illustrated in a schematically highly simplified manner.

The blood tubing set 200 comprises a hemofilter 201 or is connected thereto. A blood withdrawal line 203 (or arterial line) and a blood return line 205 (or venous line) are connected to the hemofilter 201 or dialyzer or blood filter.

The blood withdrawal line 203 is operatively connected to, or comprises, a blood pump 301.

A further addition line, here a line 207 for citrate solution, opens upstream of the blood pump 301 into the blood withdrawal line 203.

The line 207 is operatively connected to, or comprises, a citrate pump 307.

Downstream of the hemofilter 201, the line 209 for calcium solution according to the present invention opens into the blood return line 205.

The insert piece 100 according to the present invention is operatively connected to a calcium pump 309 or comprises the latter. It is supplied by a source for infusion solution which is not shown in Fig. 11, herein exemplarily a calcium source. The source may be a bag or a bottle. The infusion solution may optionally be generated on-line; in such case the respective device, in which the infusion solution is generated, is considered as a source.

The operative connection to the calcium pump 309 is to be understood herein as an example. Arranging the insert piece 100 behind another pump than a calcium pump, i.e. for example downstream of a citrate pump like the citrate pump 307 of Fig. 11, is encompassed by the present invention.

The hemofilter 201 is connected to a line 311 for fresh dialysis liquid and to a line 315 for spent dialysate or filtrate. The line 311 is connected to, or comprises, a dialysis liquid pump 313. The line 315 is connected to, or comprises, a filtrate pump 317.

The arrowhead indicates the flow direction when using the blood tubing set 200 as intended.

The blood tubing set 200 shown in Fig. 11 may correspond to a standard extracorporeal blood tubing set and may in particular be suitable for the CWHD (continuous venovenous hemodialysis).

The pumps 301, 307, 309, 313 and 317 may be part of a blood treatment apparatus 300 which is only schematically indicated. The same applies for the lines 311 and 315.

### List of reference numerals

- 100: insert piece
- 101: first connection site
- 101a: entrance
- 103: second connection site
- 103a: exit
- 105: third connection site
- 107a: first main line
- 107b: second main line
- 109: junction portion or connection portion

- 111: secondary line
- 115: outlet opening or outlet
- 119: chamfer
- 130: through-opening

- 140: upper part
- 150: lower part

- 200: blood tubing set, blood tubing system
- 201: hemofilter or blood filter or dialyzer
- 203: blood withdrawal line
- 205: blood return line
- 205a: first tubing portion
- 205b: second tubing portion
- 207: line for citrate solution
- 209: line for calcium solution; addition line; third tubing portion

- 300: blood treatment apparatus
- 301: blood pump
- 307: citrate pump
- 309: calcium pump
- 311: line for fresh dialysis liquid
- 313: pump for fresh dialysis liquid
- 315: line for spent dialysate, or filtrate
- 317: pump for spent dialysate, or filtrate

- H: flow direction of the first fluid into and through the first main line
- B: flow direction of the first fluid into and through the second main line
- N: inflow direction of the second fluid into the secondary line

## Claims

1. An insert piece (100) for a blood tubing set (200), comprising at least, respectively,
- a first connection site (101) for connecting a first tubing portion (205a) of the blood tubing set (200) to the insert piece (100);
- a second connection site (103) for connecting a second tubing portion (205b) of the blood tubing set (200) to the insert piece (100);
- a third connection site (105) for connecting a third tubing portion (209) of the blood tubing set (200) to the insert piece (100);
- a first main line (107a) for conducting a first liquid, preferably blood, through the insert piece (100), wherein the first main line (107a) is in fluid communication with the first connection site (101) and with the second connection site (103);
- a second main line (107b) for conducting a first liquid, preferably blood, through the insert piece (100), wherein the second main line (107b) is in fluid communication with the first connection site (101) and with the second connection site (103);
- a secondary line (111) for conducting a second liquid, preferably an infusion, into at least one of
the first main line (107a);
the second main line (107b); and
a connection portion (109) which connects both main lines (107a, 107b) to each other or to the second connection site (103);
wherein
- the secondary line (111) is in fluid communication with the third connection site (105);
- the first connection site (101) comprises a perfusable lumen having a first cross section area;
- the second connection site (103) comprises a perfusable lumen having a second cross section area; and
- the first main line (107a) and/or the second main line (107b) comprises a perfusable lumen having a third cross section area.

2. The insert piece (100) according to claim 1, wherein the sum of the first cross section area of the first main line (107a) and the second cross section area of the second main line (107b) is smaller than the third cross section area of the first connection site (101), the second connection site (103) and/or the cross section area of tube section of the blood tube set (200) connected to the insert piece (100).

3. The insert piece (100) according to claim 1, wherein the secondary line (111) is connected in fluid communication to, or comprises, a source for the infusion solution.

4. The insert piece (100) according to any one of the preceding claims, wherein the third cross section area is maximally half as large or substantially half as large as the first cross section area and/or the second cross section area.

5. The insert piece (100) according to any one of the preceding claims, wherein the connection portion (109) is arranged in flow direction of the first liquid between the first main line (107a) and/or the second main line (107b) on the one side, and the second connection site (103) and/or third connection site (105) on the other.

6. The insert piece (100) according to any one of the preceding claims, wherein the connection portion (109) comprises, in flow direction towards the second connection site (103), a cross section, in particular a conical one, which widens or enlarges along the flow direction.

7. The insert piece (100) according to any one of the preceding claims, wherein a through-opening (130) of the inset piece (100) is arranged between sections of the first main line (107a) and sections of the second main line (107b).

8. The insert piece (100) according to any one of the preceding claims, consisting of two or at least two components, which are welded and/or glued with or to each other.

9. The insert piece (100) according to any one of the preceding claims, wherein the first main line (107a) and/or the second main line (107b), or sections thereof, respectively, are tubes, in particular those inserted into tube intakes which are in fluid communication with the first connection site (101) or with the second connection site (103) and are integrally produced therewith.

10. The insert piece (100) according to any one of the preceding claims, wherein the first main line (107a) and/or the second main line (107b) are integral parts of the insert piece (100).

11. A blood tubing set (200) comprising at least one insert piece (100) according to any one of the preceding claims, wherein the insert piece (100) is connected in fluid communication with an addition line (207, 209) and with a blood withdrawal line (203) and/or with a blood return line (205).

12. The blood tubing set (200) according to claim 11, which is suitable and/or prepared for executing a regional anticoagulation.

13. The blood tubing set (200) according to any one of claims 11 to 12, wherein the blood tubing set (200) is suitable for executing a hemodialysis, a hemofiltration, a hemodiafiltration, a plasmapheresis treatment or a whole blood adsorption treatment.

14. The blood tubing set (200) according to any one of claims 11 to 13, wherein the first connection site (101) and the second connection site (103) are connected to a blood-return line (205) of the blood tubing set (200), and wherein the third connection site (105) is connected to a line for an electrolyte solution, in particular a calcium line (209).

15. A blood treatment apparatus (300), connected to at least a blood tubing set (200) according to any one of claims 11 to 14.
